(19) Europäisches Patentamt
European Patent Office
Office européen des brevets

(11) **EP 3 093 031 A1**

(12) **EUROPÄISCHE PATENTANMELDUNG**

(43) Veröffentlichungstag:
**16.11.2016 Patentblatt 2016/46**

(21) Anmeldenummer: **15167090.8**

(22) Anmeldetag: **11.05.2015**

(51) Int Cl.:
*A61M 1/00* *(2006.01)*        *A61M 3/02* *(2006.01)*
*A61F 13/00* *(2006.01)*

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**
Benannte Validierungsstaaten:
**MA**

(71) Anmelder: **3M Innovative Properties Company
St. Paul, MN 55133-3427 (US)**

(72) Erfinder: **Bonn, Florian
51379 Leverkusen (DE)**

(74) Vertreter: **CPW GmbH
Kasinostraße 19-21
42103 Wuppertal (DE)**

(54) **WUNDVERSORGUNGSSYSTEM**

(57)    Wundversorgungssystem zur Behandlung einer Wunde, umfassend mindestens ein erstes und ein zweites flächenförmig ausgebildetes Kapillarmembransystem,
- wobei das erste und das zweite flächenförmig ausgebildete Kapillarmembransystem mit jeweils mindestens einer Versorgungsleitung verbunden sind, so dass durch die Versorgungsleitung und das jeweilige Kapillarmembransystem Flüssigkeiten, Medien, Gase und/oder andere Stoffe durchleitbar sind,

wobei das Wundversorgungssystem des Weiteren umfasst
- einen ersten Entnahmebehälter, welcher über einen Leitungsweg, der mindestens eine der Versorgungsleitungen des ersten Kapillarmembransystems umfasst, mit dem ersten Kapillarmembransystem lösbar verbunden ist und über den dem ersten Kapillarmembransystem eine Spülflüssigkeit zuführbar ist,
- einen zweiten Entnahmebehälter, welcher über einen Leitungsweg, der mindestens eine der Versorgungsleitungen des zweiten Kapillarmembransystems umfasst, mit dem zweiten Kapillarmembransystem lösbar verbunden ist und über den dem zweiten Kapillarmembransystem eine Behandlungslösung zuführbar ist, sowie
- ein Drainagesystem, welches mit einer Unterdruckeinheit koppelbar ist und über das Flüssigkeiten aus der zu behandelnden Wunde abführbar sind.

Fig. 1

EP 3 093 031 A1

**Beschreibung**

Die Erfindung betrifft ein Wundversorgungssystem zum Einbringen in eine Wunde bzw. zum Aufbringen auf eine Hautwunde und unter einen Wundverband, umfassend mindestens ein erstes und ein zweites flächenförmig ausgebildetes Kapillarmembransystem, wobei das erste und das zweite flächenförmig ausgebildete Kapillarmembransystem mit jeweils mindestens einer Versorgungsleitung verbunden sind, so dass durch die Versorgungsleitung und das jeweilige Kapillarmembransystem Flüssigkeiten, Medien, Gase und/oder andere Stoffe durchleitbar sind.

Die moderne Wundversorgung verfolgt das Ziel, bei der Wundversorgung ein feuchtes Wundmileu zu schaffen, das bei der Heilung ablaufende Prozesse fördert. Je nach Heilungsphase müssen moderne, aktive Wundauflagen deshalb in der Lage sein, die Wunde feucht zu halten, für einen verbesserten Flüssigkeits-/Stoffaustausch, für das Einbringen von Faktoren/Medikamenten und/oder für eine verbesserte Flüssigkeits-/Sekrets- und/oder Stoffentfernung in der Wunde bzw. in die Wunde zu sorgen. Anwendungen schließen die Nutzung solcher Wundauflagesysteme in einer Weichteilwunde, in einer abdominellen Wunde und auf einer Hautwunde ein.

Ein Verfahren und eine Vorrichtung zur Entfernung von Sekret bzw. Exsudat aus Wunden ist kommerziell bekannt als V.A.C.® Therapy System (Fa. KCI, USA). Bei diesem System wird für ein alternierendes Einbringen von Flüssigkeit in die Wunde und ein nachfolgendes also auch alternierendes und damit nicht kontinuierliches Ausführen von Flüssigkeit aus der Wunde gesorgt. Ein in die Wunde eingebrachtes Schaumstoffmaterial, das bei Unterdruck Kräfte auf die Wunde ausübt, soll bei diesem System die Wundheilung begünstigen.

In der DE 10 2006 042 732 wird ein Kapillarmembransystem zur Wundbehandlung beschrieben, bei dem die Wunde über eine Hohlfasermembrananordnung aus bis zu 1000 Hohlfasern mit mindestens einer gemeinsamen Zu- und mindestens einer gemeinsamen Ableitung im Sinne der Durchströmung eines Kapillarbettes perfundiert und versorgt werden und eine Antibiotika- sowie Wachstumsfaktorenperfusion ermöglicht werden soll. Dabei soll eine gleichmäßige Stoffverteilung unter kontinuierlicher Perfusion auch unter Erzeugung eines moderaten Unterdruckes ermöglicht werden. Die DE 10 2006 042 732 führt aus, dass für eine optimale Ver- und Entsorgung weitere Kapillarmembransysteme von Vorteil sind.

Wenngleich sich bereits mit den in der DE 10 2006 042 732 beschriebenen Kapillarmembransystemen Fortschritte bei der Wundbehandlung erzielen lassen, besteht Bedarf an einfachen und effizienten Wundversorgungssystemen, mit denen zum einen die bei der Wundbehandlung notwendigen Vorgänge, wie Spülen und Desinfizieren, ohne Entfernen des Verbandes durchgeführt werden können, die erforderlichenfalls eine Ernährung, Elektrolytaustausch und/oder Detoxifikation oder auch eine Wachstumsfaktorenversorgung oder eine Versorgung mit Antibiotika ermöglichen und die eine einfache und sichere Handhabung erlauben.

Vielfach besteht der Wunsch, ein geeignetes Wundversorgungssystem zur Verfügung zu haben, das auf der einen Seite eine ausreichende Reinigung der Wunde sowie auf der anderen Seite die gezielte Applikation von Wirksubstanzen in die Wunde ermöglicht. Es ist daher Aufgabe der vorliegenden Erfindung, ein derartiges Wundversorgungssystem zur Verfügung zu stellen.

Die Aufgabe wird durch ein Wundversorgungssystem zur Behandlung einer Wunde gelöst, welches mindestens ein erstes und ein zweites flächenförmig ausgebildetes Kapillarmembransystem umfasst,

- wobei das erste und das zweite flächenförmig ausgebildete Kapillarmembransystem mit jeweils mindestens einer Versorgungsleitung verbunden sind, so dass durch die Versorgungsleitung und das jeweilige Kapillarmembransystem Flüssigkeiten, Medien, Gase und/oder andere Stoffe durchleitbar sind,

wobei das Wundversorgungssystem des Weiteren umfasst

- einen ersten Entnahmebehälter mit einem Auslass, welcher über eine erste Ablaufleitung, die mindestens eine der Versorgungsleitungen des ersten Kapillarmembransystems umfasst, mit dem ersten Kapillarmembransystem lösbar verbunden ist und über den dem ersten Kapillarmembransystem eine Spülflüssigkeit zuführbar ist,
- einen zweiten Entnahmebehälter mit einem Auslass, welcher über eine zweite Ablaufleitung, die mindestens eine der Versorgungsleitungen des zweiten Kapillarmembransystems umfasst, mit dem zweiten Kapillarmembransystem lösbar verbunden ist und über den dem zweiten Kapillarmembransystem eine Behandlungslösung zuführbar ist, sowie
- ein Drainagesystem, welches mit einer Unterdruckeinheit koppelbar ist und über das Flüssigkeiten aus der zu behandelnden Wunde abführbar sind.

Mittels eines solchen Wundversorgungssystem ist eine nahezu homogene Versorgung einer Wunde möglich, wobei die Konfiguration des Wundversorgungssystems gleichzeitig eine sichere und einfache Handhabung erlaubt. In der Anwendung kann dazu das Wundversorgungssystem in die Wunde eingelegt und z.B. mittels einer semiokklusiven transparenten Folie abgedeckt werden, um die Wunde vor dem Austrocknen oder vor Infektionen zu schützen. Die Versorgungsleitungen der Kapillarmembransysteme werden dann unter der Folie aus dem Wundbereich herausgeführt und sind außerhalb des Wundbereichs mit dem ersten bzw. dem zweiten Entnahmebehälter verbunden. Das Drainagesystem kann mit einer Unterdruckeinheit z.B. über einen geeigneten, gegen Unterdruck stabilen Schlauch in Verbindung stehen, der ebenfalls aus dem Wundbereich herausgeführt wird.

Hierbei kann das erste bzw. das zweite flächenförmig ausgebildete Kapillarmembransystem aus einer einzelnen Kapillarmembran bestehen, die mäanderförmig angeordnet ist. Bei dieser Ausführungsform ist mindestens eines der Enden der mäanderförmigen Kapillarmembran offen und mit einer Versorgungsleitung verbunden. Das mindestens eine Kapillarmembransystem kann jedoch auch mehrere mäanderförmig angeordnete Kapillarmembranen umfassen, die zusammen mit ihren Enden in eine gemeinsame Versorgungsleitung münden. Vorzugsweise umfasst das erste bzw. das zweite Kapillarmembransystem eine Mehrzahl von zueinander parallel angeordneten Kapillarmembranen.

Die zueinander parallel angeordneten Kapillarmembranen eines jeweiligen Kapillarmembransystems sind an mindestens einem ihrer Enden so an ihrem äußeren Umfang fluiddicht in der Wand einer Versorgungsleitung eingebettet, dass zwischen dem Lumen der Versorgungsleitung und dem Lumen der Kapillarmembranen eine Fluidverbindung besteht und durch die Versorgungsleitung und das mindestens eine Kapillarmembransystem Flüssigkeiten, Medien, Gase und/oder andere Stoffe durchleitbar sind. Die Einbettung kann beispielsweise mit einem härtbaren Silikonmaterial, mit einem Polyurethan- oder einem Epoxidharz erfolgen. Bevorzugt werden wegen ihrer besseren Flexibilität härtbare Silikonmaterialien eingesetzt. Im Falle, dass die Kapillarmembranen nur mit einem ihrer Enden in einer Versorgungsleitung eingebettet sind, ist das andere, gegenüberliegende Ende der Kapillarmembranen geschlossen, beispielsweise durch Verschweißen oder Verkleben. Die Kapillarmembranen können auch an ihren beiden Enden offen und mit diesen beiden Enden auf einer Seite der Anordnung in eine einzelne Versorgungsleitung eingebettet sein, wobei die Kapillarmembranen dann an ihrem freien Ende U-förmig ausgebildet sind und dadurch dort geschlossen sind. In diesen Fällen werden die Kapillarmembranen im Dead-end Modus betrieben.

Insbesondere bei breiteren Wundversorgungssystemen sind Ausführungsform der Kapillarmembransysteme mit parallel zueinander angeordneten Kapillarmembranen von Vorteil, bei dem die Kapillarmembranen an ihren beiden Enden offen und in jeweils eine Versorgungsleitung eingebettet sind, wobei sich die Versorgungsleitungen sich dann bevorzugt an gegenüberliegenden Seiten der des jeweiligen flächenförmig ausgebildeten Kapillarmembransystems befinden. Auch in diesem Fall ist die Einbettung so ausgeführt, dass die Kapillarmembranen an ihrem äußeren Umfang fluiddicht eingebettet sind und zwischen dem Lumen der jeweiligen Versorgungsleitung und dem Lumen der Kapillarmembranen eine Fluidverbindung besteht. Eine derartige Ausführungsform mit zwei Versorgungsleitungen erlaubt eine Versorgung und/oder Entsorgung über das mindestens eine Kapillarmembransystem im cross-flow Modus. Auch mit Blick auf eine gute Homogenität der Versorgung bzw. Entsorgung über der Fläche der Wunde kann insbesondere bei breiteren Matten bzw. Wundversorgungssystemen die Ausführung des mindestens einen Kapillarmembransystems mit zwei Versorgungsleitungen zweckmäßig sein. Eine Ausführungsform mit zwei Versorgungsleitungen ermöglicht jedoch auch eine gleichzeitige oder auch eine alternierende Versorgung einer Wunde mit unterschiedlichen Medien über dasselbe Kapillarmembransystem.

Der Durchmesser der Versorgungsleitungen richtet sich in erster Linie nach dem Außendurchmesser der in sie eingebetteten Kapillarmembranen. Daher weist die mindestens eine Versorgungsleitung bevorzugt einen Innendurchmesser im Bereich von 0,1 bis 10 mm auf. Ebenso ist bevorzugt, wenn die Wandstärke im Bereich von 0,1 bis 5 mm liegt. Im Falle der Verwendung einer Versorgungsleitung mit nicht-kreisförmigem Querschnitt wird als Innendurchmesser der äquivalente Durchmesser $d = 4A/U$ des Innenquerschnitts angesetzt mit A als der Fläche des Innenquerschnitts und U als dessen Umfang. Beispielsweise kann die Versorgungsleitung auch einen ovalen, oder näherungsweise quadratischen oder rechteckigen Innenquerschnitt aufweisen. Für die Versorgungsleitungen haben sich beispielsweise Silikonschläuche als geeignet erwiesen, durch deren Wand die Kapillarmembranenden hindurchtreten und in der sie eingeklebt sind. Vorzugsweise sind die Versorgungsleitungen aus einem flexiblen Silikonschlauch ausgebildet. Die Einbettung bzw. das Einkleben in die Wand der Versorgungsleitung kann mittels üblicher Kleber wie z.B. mittels härtbarer Silikonmaterialien, Polyurethanharzen oder einem Epoxidharzen erfolgen.

In einer vorteilhaften Ausführungsform kann das erste und/oder das zweite Kapillarmembransystem in Form einer Kapillarmembranmatte aus parallel zueinander angeordneten Kapillarmembranen ausgebildet sein, wobei die Kapillarmembranen in der Matte mittels zueinander beabstandeter und zueinander parallel verlaufender Verbindungselemente miteinander verbunden und durch die Verbindungselemente zueinander auf Abstand gehalten sind. Die Verbindungselemente können quer zu den parallel zueinander angeordneten Kapillarmembranen verlaufen oder auch unter einem anderen Winkel. Dabei berühren die Verbindungselemente die Kapillarmembranen an ihrem äußeren Umfang oder die umschlingen diese. Die Verbindungselemente weisen entlang ihrer Längserstreckung keine geschlossenen Strömungskanäle auf, sind also folglich entlang ihrer Längserstreckung nicht von Fluiden durchströmbar. Bei den Verbindungselementen kann es sich um Klebestreifen handeln oder beispielsweise auch um strangförmige Elemente aus einem Silikonmaterial. In einer bevorzugten Ausführungsform sind die Kapillarmembranen mittels garnförmiger Verbindungselemente zu einer Matte verbunden. Besonders bevorzugt handelt es sich bei den Verbindungselementen um textile Multifilamentgarne. Besonders bewährt haben sich als textile Multifilamentgarne multifile Polyestergarne, Polypropylengarne oder Polytetrafluorethylengarne. Bestens geeignet sind hydrophile Garne, vorzugsweise aus Polyester.

Bei der Kapillarmembranmatte kann es sich in einer bevorzugten Ausführungsform um eine Wirkmatte handeln. Bei solchen Wirkmatten sind die Kapillarmembranen und die Verbindungsfäden miteinander verwirkt und die Kapillarmembranen verlaufen quer zur Erstreckungsrichtung der Kapillarmembranmatte. Die Länge der Kapillarmembranen ist durch die Mattenbreite bestimmt. In einer weiteren bevorzugten Ausführungsform kann es sich bei der Kapillarmembranmatte um eine Webmatte handeln. Bei solchen Webmatten sind die Kapillarmembranen und die Verbindungsfäden miteinander verwoben. Die Kapillarmembranen verlaufen dabei in Erstreckungsrichtung oder Laufrichtung der Kapillarmembranmatte und die textilen Fäden quer dazu. Kapillarmembranwirkmatten und -webmatten sowie Möglichkeiten ihrer Herstellung werden beispielsweise in der DE 38 39 567, der DE 43 08 850 und in der EP 0 442 147 beschrieben. Insbesondere mittels der Wirktechnologie lassen sich auf einfache Weise Matten herstellen, bei denen die Kapillarmembranen an ihrem freien Ende U-förmig ausgebildet und dort verschlossen sind. Derartige Matten können durch mäanderförmige Ablage einer Kapillarmembran in zueinander parallele Stränge, die durch die Wirkfäden miteinander verbunden sind, hergestellt werden. Dabei werden nach Fertigstellung der Wirkmatte die U-förmig ausgebildeten Enden an mindestens einer Seite der Wirkmatte abgetrennt und die dabei entstehenden offenen Enden der Kapillarmembranen dann einer Versorgungsleitung eingebettet. Im Falle, dass die U-förmig ausgebildeten Enden an beiden Seite der Wirkmatte abgetrennt werden, können die entstehenden gegenüberliegenden offenen Enden jeweils in Versorgungsleitungen eingebettet werden.

In einer bevorzugten Ausgestaltung liegen die Kapillarmembranen innerhalb der Matte in einer solchen Dichte vor, dass der Abstand der Kapillarmembranen zueinander in der Matte das 1 bis 10-fache des Außendurchmessers der Kapillarmembranen beträgt, wobei der Abstand von den Längsachsen der Kapillarmembranen gemessen wird Dabei sind Matten bevorzugt, in denen der Abstand der Kapillarmembranen zueinander in der Matte das 1,05 bis 6-fache des Außendurchmessers der Kapillarmembranen beträgt. Besonders bevorzugt sind Abstände der Kapillarmembranen zueinander in der Matte im Bereich des 1,05 bis 3-fachen des Außendurchmessers der Kapillarmembranen. In einer weiteren besonders bevorzugten Ausführungsform sind dabei Abstände der Kapillarmembranen zueinander in der Matte von mehr als dem 1,5 fachen des Außendurchmessers der Kapillarmembranen. Es wurde gefunden, dass sich hiermit eine sichere Trennung der Kapillarmembranen voneinander erreichen lässt.

Gleichzeitig kann es auch im Hinblick auf eine gute homogene Versorgung der zu behandelnden Wunde wichtig sein, dass die Kapillarmembranen in den Kapillarmembransystemen mittels mehrerer zueinander beabstandeter und zueinander parallel verlaufender Verbindungselemente miteinander zu einer Matte verbunden und durch die Verbindungselemente zueinander auf Abstand gehalten sind, und dass die Verbindungselemente sich zueinander in einem definierten Abstand befinden, der vorzugsweise im Bereich von 1 bis 50 mm liegt, wobei ein Abstand im Bereich von 3 bis 20 mm besonders bevorzugt und ein solcher im Bereich von 4 bis 6 mm bestens geeignet ist. Es hat sich nämlich gezeigt, dass die Kontaktstellen zwischen den Kapillarmembranen und den Verbindungselementen z.B. bei einer Versorgung der zu behandelnden Wunde z.B. mit einer Behandlungslösung oder einer Nährlösung in erheblichem Maße Verteilung der Flüssigkeit über der Fläche der Anordnung der Kapillarmembranen fördern. So wurde bei Auflage solcher Kapillarmembransysteme auf zu behandelnde Wunden beobachtet, dass es an den Kontaktstellen zu einer Begünstigung des Austritts von Flüssigkeit aus den Kapillarmembranen kommt.

Die Kapillarmembranen der Kapillarmembransysteme weisen bevorzugt einen Außendurchmesser im Bereich von 200 bis 1500 $\mu$m auf. Ebenso sind Kapillarmembranen mit einer Wandstärke im Bereich von 20 bis 400 $\mu$m von Vorteil, wobei deren Außendurchmesser vorzugsweise in den zuvor genannten Bereichen liegen kann.

Bei dem vorliegenden Wundversorgungssystem sind erstes und zweites Kapillarmembransystem für die Zuführung bzw. Abführung von flüssigen Medien ausgelegt. Um dann eine gleichmäßige Versorgung der zu behandelnden Wunde zu gewährleisten, weisen in einer bevorzugten Ausführungsform die Kapillarmembranen eine hohe Permeabilität für Flüssigkeiten auf. Vorzugsweise liegt hierbei der Transmembranfluss für Wasser der Kapillarmembranen im Bereich von 0,01 bis 50 ml/(min·cm$^2$·bar).

Die Kapillarmembranen des ersten und/oder zweiten Kapillarmembransystems sind vorzugsweise bakteriendicht. Hierdurch kann gewährleistet werden, dass durch eine Zuführung von Spülflüssigkeit und/oder Behandlungslösung keine Bakterien in die Wunde gelangen. Dabei wird im Rahmen der vorliegenden Erfindung unter Bakteriendichtigkeit verstanden, dass die Kapillarmembranen eine nominelle Pore von 0,2 $\mu$m aufweisen. Bevorzugt weisen also die Kapillarmembranen des ersten und/oder des zweiten Kapillarmembransystems eine nominelle Pore von 0,2 $\mu$m auf. Dabei wird die nominelle Pore über das Rückhaltevermögen der Membran gegenüber spezifischen Mikroorganismen definiert. So hält beispielsweise eine Membran mit einer nominellen Pore von 0,2 $\mu$m Bakterien der Gattung Brevundimonas diminuta zurück, aber auch Bakterien der Gattung Serratia marcescens, für die eine Membran mit nomineller Pore von 0,45 $\mu$m ausreichend wäre. Die Prüfungen bzw. die Ermittlung der nominellen Porengrößen sind beispielsweise in der HIMA-Vorschrift No. 3, Vol. 4, 1982 (Health Industry Manufacturers Association) beschrieben.

Als Materialien für die Kapillarmembranen kommen grundsätzlich alle im Stand der Technik bekannten organischen Polymere in Frage, die zur Ausbildung von Kapillarmembranen geeignet sind, wobei diese Polymere eine gute Biokompatibilität aufweisen müssen. Darüber hinaus ist es auch erforderlich, dass das Membranpolymer eine Sterilisation des Wundversorgungssystems beispielsweise über Dampfsterilisation, Sterilisation mittels $\gamma$-Strahlung oder Sterilisation mittels Ethylenoxid erlaubt. Dabei können die organischen Polymere natürliche Polymere sein oder Polymere, die auf synthetischen Wege hergestellt wurden. Natürliche Polymere sind insbesondere solche auf Basis von zellulosischen Polymeren, was Polymere, die sog. polymeranalogen Reaktionen unterzogen worden sind, ebenfalls umfasst. Beispiele für Polymere auf Basis von Zellulose sind solche aus regenerierter Zellulose, Zelluloseazetat oder modifizierter Zellulose wie z.B. Zelluloseester, Zelluloseäther, mit Benzylgruppen modifizierte Zellulose (Benzylzellulose) oder mit Diethylaminoethyl modifizierte Zellulose oder Mischungen dieser zellulosischen Polymere. Des Weiteren können auch Polymere auf der Basis von Chitin, bzw. Chitosan zum Einsatz kommen.

Als auf synthetischem Wege hergestellte Polymere, d.h. als synthetische Polymere können solche verwendet werden, die aus Polyolefinen, Polyamiden, Polyacrylnitrilen, Polycarbonaten, Polyestern oder Sulfonpolymeren sowie daraus gewonnen Modifikationen, Blends, Mischungen oder Copolymere dieser Polymere bestehen. Vorzugsweise werden solche verwendet, die auf Sulfonpolymeren, wie insbesondere Polysulfon oder Polyethersulfon, basieren. Den synthetischen Polymeren können weitere Polymere wie z.B. Polyethylenoxid, Polyhydroxyether, Polyethylenglykol, Polyvinylalkohol oder Polycaprolacton als Zusatzstoffe beigemischt werden. Die Kapillarmembranen können darüber hinaus noch eine Beschichtung mit einem Additiv aufweisen. Bevorzugt enthalten solche Kapillarmembranen ein Hydrophilierungsmittel, z.B. Polyvinylpyrrolidon oder auch hydrophile Modifikationen dieser Polymere.

Die Kapillarmembranen können mit Blick auf bestimmte Anwendungen z.B. über Ankopplung funktioneller Gruppen modifiziert sein oder beispielsweise mit Heparin oder einem Antibiotikum oder mehreren Antibiotika beschichtet sein.

Die Form der flächenförmig ausgebildeten Kapillarmembransysteme in ihrer flächigen Erstreckung kann beliebig sein. Im Fall von Kapillarmembransystemen aus zueinander parallelen Kapillarmembranen weisen die Kapillarmembransysteme in der einfachsten Ausführung eine quadratische oder rechteckige Form auf. Es ist jedoch z.B. bei Systemen, bei denen die Kapillarmembranen nur an einem ihrer Enden in eine Versorgungsleitung eingebettet sind, möglich, dass beispielsweise durch entsprechend angepasstes Abschweißen der freien, verschlossenen Enden der zueinander parallelen Kapillarmembranen eine bogenförmige Kontur ausgebildet wird. Ebenso ist es möglich, dass die Anordnung aus zueinander parallelen Kapillarmembranen z.B. auch eine trapezförmige Kontur aufweist.

Das vorliegende Wundversorgungssystem kann auch weitere Komponenten aufweisen wie z.B. mindestens eine weitere Anordnung von Kapillarmembranen. Bei den Kapillarmembranen der weiteren Anordnung kann es sich beispielsweise um Membranen zur Oxygenation handeln, d.h. Membranen, über die eine Zuführung von Sauerstoff zur Wunde möglich ist. Derartige Membranen werden beispielweise in der in der EP-A-1 144 096, der EP-A-0 299 381 oder der DE-A-28 33 493 offenbart. Auch eine Kombination mit weiteren flächenförmig ausgebildeten Systemen bzw. Anordnungen von semipermeablen Kapillarmembranen oder fluidundurchlässigen Kapillaren ist möglich, über die z.B. eine Temperierung oder eine pH-Wert Regulierung erfolgen kann. Dabei können die jeweiligen Kapillarmembransysteme und eventuelle weitere flächenförmig ausgebildete Systeme von semipermeablen Kapillarmembranen oder fluidundurchlässigen Kapillaren aufeinander gelegt werden. Es ist jedoch auch möglich, dass z.B. die Kapillarmembranen unterschiedlicher Kapillarmembransysteme miteinander zu einer Matte verbunden sind, wobei die unterschiedlichen Kapillarmembranen mit ihren Enden in unterschiedliche Versorgungsleitungen eingebettet sind, die vorzugsweise an entgegengesetzten Seiten der Matte angeordnet sind. Solche Matten können beispielsweise durch Verwirken von zueinander versetzt angeordneten, mäanderförmig abgelegten Kapillarmembranen erhalten werden, bei denen die U-förmigen Umlenkungen der Kapillarmembranen sich an unterschiedlichen Positionen über der Mattenbreite befinden. Durch Schneiden der jeweils außenliegenden U-förmigen Umlenkungen werden die Kapillarmembranen an jeweils nur einer Seite der Matte geöffnet und können dort in eine Versorgungsleitung eingebettet werden.

Erfindungsgemäß umfasst das Wundversorgungssystem neben dem ersten und dem zweiten Kapillarmembransystem ein Drainagesystem, mittels dessen eine Abführung z.B. der Spülflüssigkeit oder von Exsudat aus der Wunde möglich ist. In einer Ausführungsform kann das Wundversorgungssystem einen Saugschwamm aufweisen, welcher über eine Absaugleitung für die Spülflüssigkeit und/oder für Exsudat verfügt. Das Drainagesystem kann auch als ein weiteres Kapillarmembransystem ausgebildet sein. Vorzugsweise handelt es sich bei dem Drainagesystem jedoch um mindestens einen Drainagekatheter, z.B. in Gestalt eines Schlauchstücks, beispielsweise aus einem Silikonmaterial, oder eines Röhrchens. Ein solcher Drainagekatheter kann in seiner Wand Perforierungen aufweisen, über die nach Anschluss des Drainagekatheders an eine Unterdruckeinheit Flüssigkeiten aus der Wunde abgesaugt werden können. Der mindestens eine Drainagekatheter weist bevorzugt einen Innendurchmesser im Bereich von 0,1 bis 15 mm und eine Wandstärke im Bereich von 0,1 bis 3 mm auf. Der Drainagekatheter kann auch einen nicht-kreisförmigen Querschnitt aufweisen. In diesem Fall wird als Innendurchmesser der äquivalente Durchmesser $d_D = 4A_D/U_D$ des Innenquerschnitts angesetzt mit $A_D$ als der Fläche des Innenquerschnitts des Drainagekatheters und $U_D$ als dessen Umfang.

In einer Ausführungsform kann das Wundversorgungssystem des Weiteren eine taschenförmige Wundauflage umfassen, wobei die taschenförmige Wundauflage an ihrem äußeren Rand geschlossen ist und eine Oberseite, eine Unterseite und ein Tascheninneres aufweist, wobei die Unterseite und die Oberseite jeweils aus einem flächigen Material ausgebildet sind und die Unterseite permeabel für Fluide ist, und wobei erstes und zweites Kapillarmembransystem im Tascheninneren angeordnet sind. Hierbei liegt die Verbindung der Versorgungsleitungen mit den jeweiligen Aufnahmebehältern außerhalb der taschenförmigen Wundauflage.

Die taschenförmige Wundauflage mit den darin enthaltenen Kapillarmembransystemen kann so in eine zu behandelnde Wunde eingelegt werden, dass die Unterseite mit der Wunde in Kontakt ist. Über die Kapillarmembransysteme können der Wunde die gewünschten Flüssigkeiten zugeführt werden, die sich nach Austritt aus den Kapillarmembranen in der Tasche verteilen und über die semipermeable Taschenunterseite an die Wunde abgegeben werden.

Die taschenförmige Wundauflage kann vorzugsweise so ausgeführt sein, dass sich die Verbindung der Kapillarmembranen der Kapillarmembransysteme mit der jeweiligen Versorgungsleitung im Tascheninneren befindet und die jeweiligen Versorgungsleitungen über an ihren äußeren Querschnitt fluiddicht angepasste Durchtrittsöffnungen aus der taschenförmigen Wundauflage herausführen. Ebenso kann die Verbindung der Kapillarmembranen mit der jeweiligen Versorgungsleitung außerhalb der taschenförmigen Wundauflage auf der Oberseite angeordnet sein und die Anordnung der Kapillarmembranen zur Verbindung mit der mindestens einen Versorgungsleitung über eine fluiddicht angepasste Durchtrittsöffnung aus der taschenförmigen Wundauflage herausführen.

Im Falle, dass das Wundversorgungssystem eine taschenförmigen Wundauflage umfasst, in der erstes und zweites Kapillarmembransystem angeordnet sind, erstrecken sich die Kapillarmembransysteme flächig im Tascheninneren. Die Dimensionen der Kapillarmembransysteme ergeben sich jeweils aus deren äußeren Abmessungen in der flächigen Erstreckung. Vorzugsweise füllen erstes bzw. zweites Kapillarmembransystem hinsichtlich ihrer flächigen Erstreckung das Tascheninnere der taschenförmigen Wundauflage in dessen flächiger Erstreckung mindestens zu 20 % und besonders bevorzugt mindestens zu 50 % aus. Von besonderem Vorteil ist es, wenn die erstes bzw. zweites Kapillarmembransystem hinsichtlich ihrer flächigen Erstreckung das Tascheninnere der taschenförmigen Wundauflage in dessen flächiger Erstreckung mindestens zu 70 % ausfüllt, wobei auch Füllgrade im Bereich von 90 % realisiert werden können. Dabei ist es von Vorteil, wenn erstes und zweites Kapillarmembransystem in der taschenförmigen Wundauflage mittig angeordnet sind.

Die taschenförmige Wundauflage kann beliebige Konturen aufweisen. Vorzugsweise ist die Kontur jedoch rund, oval, quadratisch oder rechteckig. Unterseite und Oberseite der taschenförmigen Wundauflage sind am äußeren Rand bzw. an den äußeren Kanten der Wundauflage beispielsweise durch Verschweißen oder Verkleben miteinander verbunden. Zur Verklebung sind u.a. Silikonstreifen geeignet, die ausgehärtet werden. Bei rechteckigen oder bei quadratischen taschenförmigen Wundauflagen weisen die darin angeordneten flächenförmig ausgebildeten Kapillarmembransysteme vorzugsweise ebenfalls eine rechteckige oder quadratische Kontur auf. Bei runden oder ovalen taschenförmigen Wundauflagen sind die darin befindlichen Kapillarmembransysteme zweckmäßigerweise ebenfalls quadratisch oder rechteckig ausgebildet, wobei hinsichtlich der Abmessungen ebenfalls die zuvor genannten Dimensionen gelten. Sie können jedoch auch an die Kontur der taschenförmigen Wundauflage angepasst sein, beispielsweise durch entsprechend angepasstes Abschweißen der nicht eingebetteten Enden der Kapillarmembranen bei Kapillarmembransystemen mit jeweils nur einer Versorgungsleitung, so dass sich an dieser Kante der Kapillarmembransysteme eine bogenförmige Kontur ergibt.

Die Unterseite der taschenförmigen Wundauflage ist gegenüber Fluiden durchlässig, d.h. permeabel. Dabei kann die Unterseite beispielsweise aus einem vliesförmigen, flächigen Material, einem gitterförmigen oder netzartigen Material, einer perforierten Folie oder einer semipermeablen mikroporösen Flachmembran bestehen. In einer vorteilhaften Ausführungsform besteht die Unterseite aus einem vliesförmigen, flächigen Material oder einer semipermeablen mikroporösen Flachmembran. Die Unterseite weist bevorzugt eine Permeabilität für Wasser von mindestens 0,01 ml/(min·cm$^2$·bar) und besonders bevorzugt von mindestens 10 ml/(min·cm$^2$·bar) auf. Bestens bewährt hat sich eine Unterseite mit einer Permeabilität für Wasser von mindestens 500 ml/(min·cm$^2$·bar).

Für die vorgesehenen Anwendungen des Wundversorgungssystems, bei denen der Wunde über das Wundversorgungssystem nicht nur Flüssigkeit zugeführt wird, sondern auch eine Entsorgung, d.h. Abführung von Flüssigkeiten aus der Wunde über das Drainagesystem erfolgen soll, ist es von Vorteil, wenn in der Unterseite Öffnungen vorhanden sind, wobei die Öffnungen vorzugsweise einen Durchmesser von mindestens 100 μm aufweisen. Dabei sind Durchmesser der Öffnungen von höchstens 10 mm bevorzugt und von höchstens 5 mm besonders bevorzugt. Im Falle, dass die Unterseite aus einer semipermeablen mikroporösen Flachmembran besteht, weist diese in einer vorteilhaften Ausführungsform zusätzlich Öffnungen z.B. in Form von Perforationen auf. Im Falle, dass die Öffnungen eine nicht-kreisförmige Kontur aufweisen, wird als Durchmesser der äquivalente Durchmesser D=4A/U der Öffnung angesetzt mit A als der Fläche der jeweiligen Öffnung und U als deren Umfang. Die Öffnungen können regelmäßig oder unregelmäßig über die Fläche der Unterseite verteilt sein, wobei eine regelmäßige, homogene Verteilung bevorzugt ist. Dabei kann der Abstand zwischen den Öffnungen im Bereich von 1 bis 20 mm liegen, gemessen von äußeren Rand der Öffnungen.

Unter- und die Oberseite der taschenförmigen Wundauflage können aus gleichen oder unterschiedlichen Materialien bestehen. Während die Unterseite jedoch stets gegenüber Flüssigkeiten durchlässig ist, ist die Oberseite bevorzugt aus einem fluidundurchlässigen, vorzugsweise folienförmigen Material ausgebildet, das an seiner bzw. seinen Seitenkanten fluiddicht mit der Unterseite verbunden ist. Es kann sich bei der Oberseite auch um eine semipermeable, mikroporöse Flachmembran handeln. In diesem Fall weist die Oberseite jedoch eine geringere Permeabilität gegenüber Fluiden auf als die Unterseite, um in der Anwendung eine Verteilung zugeführter Flüssigkeit auf der Unterseite der taschenförmigen Wundauflage und damit zur Wunde hin zu gewährleisten. Im Falle, dass es sich bei Unterseite und Oberseite um dieselbe oder die gleiche semipermeablen mikroporösen Flachmembran handelt, weist die Unterseite Perforationen auf.

Als Materialien für die Unter- bzw. die Oberseite der taschenförmigen Wundauflage kommen grundsätzlich dieselben organischen Polymere in Frage, die zuvor als Polymere für die Kapillarmembranen genannt wurden und die sich zu Flachfolien bzw. Flachmembranen verarbeiten lassen. Vorzugsweise sind Unter- und/oder Oberseite der taschenförmigen Wundauflage aus Polyolefinen, Polyamiden, Polyacrylnitril, Polycarbonaten Polyester oder Sulfonpolymeren sowie daraus gewonnen Modifikationen, Blends, Mischungen oder Copolymere dieser Polymere aufgebaut. Besonders bevorzugt umfassen Unter- und Oberseite als Material Sulfonpolymere, wobei Polysulfon oder Polyethersulfon bestens geeignet sind.

Das Drainagesystem, vorzugsweise in Gestalt mindestens eines Drainagekatheters, führt im bevorzugten Fall, dass das Wundversorgungssystem eine taschenförmige Wundauflage umfasst, über eine entsprechend fluiddicht angepasste Durchtrittsöffnung aus der taschenförmigen Wundauflage heraus und ist außerhalb der taschenförmigen Wundauflage mit einer Unterdruckeinheit verbindbar, um so in der Anwendung einen Unterdruck im Tascheninneren zu erzeugen. Bei dem mindestens einen Drainagekatheder kann es sich um ein Schlauchstück, beispielsweise aus einem Silikonmaterial, oder um ein Röhrchen handeln, das im Tascheninneren der Wundauflage angeordnet ist und über die Durchtrittsöffnung aus der Wundauflage herausführt. An dem sich im Inneren der taschenförmigen Wundauflage befindlichen Segment des mindestens einen Drainagekatheters weist dieser vorzugsweise in seiner Wand Perforierungen auf, über die nach Anschluss des mindestens einen Drainagekatheders an eine Unterdruckeinheit Flüssigkeiten wie z. B. auch Exsudat aus der Wunde bzw. aus dem Inneren der taschenförmigen Wundauflage und aus der Wunde abgesaugt werden kann.

In einer bevorzugten Ausführungsform sind erstes und zweites Kapillarmembransystem des Wundversorgungssystems identisch und bilden ein einziges Versorgungs-Kapillarmembransystem aus, welches mit mindestens einer Versorgungsleitung verbunden ist, und der erste Entnahmebehälter und der zweite Entnahmebehälter sind über die mindestens eine Versorgungsleitung des Versorgungs-Kapillarmembransystems mit dem Versorgungs-Kapillarmembransystem verbunden. Dabei kann das Versorgungs-Kapillarmembransystem zwei Versorgungsleitungen aufweisen, die sich an den gegenüberliegenden Enden seiner Kapillarmembranen befinden. In diesem Fall kann z.B. der erste Entnahmebehälter für Spülflüssigkeit und der zweite Entnahmebehälter mit Behandlungslösung mit unterschiedlichen und voneinander getrennten Versorgungsleitungen verbunden sein. Es ist jedoch auch möglich, dass der erste Entnahmebehälter für Spülflüssigkeit und der zweite Entnahmebehälter mit Behandlungslösung über z.B. mittels eines T-Stücks oder Y-Konnektors miteinander verbundene Leitungsabschnitte mit nur einer Versorgungsleitung des Versorgungs-Kapillarmembransystems verbunden sind. Hierbei sind auch Ausführungen des Versorgungs-Kapillarmembransystems möglich, bei dienen das Versorgungs-Kapillarmembransystem mit nur einer Versorgungsleitung verbunden ist.

Für die Anwendung ist bevorzugt, wenn die Spülflüssigkeit aus dem ersten Entnahmebehälter und die Behandlungslösung aus dem zweiten Entnahmebehälter getrennt voneinander und alternierend zugeführt werden können. Bevorzugt weist hierzu die Ablaufleitung zwischen dem ersten Entnahmebehälter und dem mit dem ersten Entnahmebehälter verbundenen Kapillarmembransystem ein dem ersten Entnahmebehälter zugeordnetes erstes Regelorgan auf. Ebenso weist die Ablaufleitung zwischen dem zweiten Entnahmebehälter und dem mit dem zweiten Entnahmebehälter verbundenen Kapillarmembransystem ein dem zweiten Entnahmebehälter zugeordnetes zweites Regelorgan auf, wobei erstes und zweites Regelorgan unabhängig voneinander einstellbar sind. Je nach Ausführung des Wundversorgungssystems können die Regelorgane am jeweiligen Entnahmebehälter, in den Ablaufleitungen zwischen den Entnahmebehältern und einem die Ablaufleitungen der Entnahmebehälter verbindenden T-Stück oder Y-Konnektor oder in der jeweiligen Versorgungsleitung angebracht sein. Die Regelorgane können in einer Ausführungsform ein Absperrorgan sein, mittels dessen die jeweilige Ablaufleitung geöffnet oder geschlossen werden kann. Es kann sich jedoch auch um Regelorgane handeln, mittels dessen der Durchfluss durch die Ablaufleitung auf definierte Werte eingeregelt werden kann.

In einer bevorzugten Ausführungsform sind erster und zweiter Entnahmebehälter über Male-/Female-Konnektoren lösbar mit der jeweiligen Versorgungsleitung verbunden. Besonders bevorzugt sind Male-/Female-Konnektoren Luer-Lock-Konnektoren. Hierbei kann mit dem jeweiligen Entnahmebehälter auch ein Schlauchabschnitt verbunden sein, der wiederum mit der jeweiligen Versorgungsleitung verbunden ist. Die Verbindung zwischen dem jeweiligen Entnahmebehälter und der Versorgungsleitung, d.h. z.B. zwischen Entnahmebehälter und Versorgungsleitung, zwischen Schlauchabschnitt und Versorgungsleitung oder zwischen Teilabschnitten des Schlauchabschnitts kann auch als sterile Schweißverbindung ausgeführt sein, wie sie mittels eines Sterilkonnektors (z.B. TSCD® II Sterile Tubing Welder, Fa. Terumo) hergestellt werden kann.

In einer weiteren vorteilhaften Ausführungsform besteht der erste und/oder der zweite Entnahmebehälter aus einer Mehrzahl von ersten Teilentnahmebehältern, die in Parallelschaltung zueinander angeordnet sind. Die Auslässe der Teilentnahmebehälter sind jeweils mit einer Teilablaufleitung verbunden, die über ein Verbindungselement und eine daran angeschlossene Verbindungsleitung mit der Versorgungsleitung des ersten bzw. des zweiten Kapillarmembransystems verbunden sind. Dabei weisen die Teilablaufleitungen vorzugsweise jeweils ein Regelorgan auf, mittels dessen der jeweilige Teilentnahmebehälter zu- bzw. abgeschaltet oder mittels dessen der Durchfluss durch die Teilablaufleitung auf definierte Werte eingestellt werden kann. Eine solche Ausführungsform ist z.B. insbesondere dann von Vorteil, wenn der Wunde z.B. Eigenserum zugeführt werden soll, das als Aliquots auf mehrere Teilentnahmebehälter aufgeteilt ist.

Wie ausgeführt, ist es bei Wundbehandlungen oftmals erforderlich, dass sich Phasen, in denen eine Spülung der Wunde erfolgt, mit Phasen abwechseln, in denen eine Applikation einer Behandlungslösung in den Wundbereich erfolgt. Hierzu umfasst das vorliegende Wundbehandlungssystem einen ersten Entnahmebehälter für eine Spülflüssigkeit und einen zweiten Entnahmebehälter für eine Behandlungslösung. In der Regel werden die Behandlungslösungen nur in geringen Mengen dem Wundbereich zugeführt, während zur ausreichenden Reinigung der Wunde und zur Entfernung von Abbauprodukten größere Volumina an Spüllösung benötigt werden. Daher beträgt in einer bevorzugten Ausführungsform das Verhältnis des Volumens des ersten Entnahmebehälters zum Volumen des zweiten Entnahmebehälters mindestens 5. Besonders bevorzugt beträgt das Verhältnis mindestens 10 und ganz besonders bevorzugt mindestens 20. Die absoluten Volumina des ersten und des zweiten Entnahmebehälters hängen u.a. von der Größe der zu behandelnden Wunde ab.

Als Spülflüssigkeit können übliche Flüssigkeiten in Frage, die für die Wundreinigung geeignet sind. Bevorzugt enthält der erste Entnahmebehälter eine Kochsalz-Lösung. Als Behandlungslösung kommen Lösungen mit Wachstumsfaktoren, Antibiotika oder andere Medikamente enthaltende Lösungen, Lösungen zur pH-Wert Regulierung oder auch Eigen- oder Fremdserum in Frage. In einer bevorzugten Ausführungsform enthält der zweite Entnahmebehälter ein Serum.

Erster und/oder zweiter Entnahmebehälter können in einer bevorzugten Ausgestaltung mit Druck beaufschlagbar sein. In der Anwendung kann es jedoch auch ausreichend sein, den ersten und/oder zweiten Entnahmebehälter über eine geeignete Haltevorrichtung in einem definierten senkrechtem Abstand über der Wunde anzuordnen, so dass das Ausfließen aus dem ersten und/oder zweiten Entnahmebehälter und das Einbringen der Spülflüssigkeit und/oder der Behandlungslösung unter dem Einfluss der Schwerkraft erfolgt.

Für die Charakterisierung der Eigenschaften der im Wundversorgungssystem eingesetzten Kapillarmembranen bzw. Flachmembranen werden die folgenden Messmethoden zu Grunde gelegt:

Transmembranfluss (Wasserpermeabilität) für Kapillarmembranen:

Aus den zu prüfenden Kapillarmembranen wird eine Prüfzelle mit definierter Kapillarmembranzahl und Länge gefertigt. Die Kapillarmembranen werden dafür beidseitig an ihren Enden in ein Polyurethanharz eingebettet. Nach dem Aushärten des Harzes werden die Einbettungen auf eine Länge von ca. 30 mm geschnitten, wobei die Lumina der Kapillarmembranen durch den Schnitt geöffnet werden. Die Kapillarlumina in den Einbettungen müssen auf Durchgängigkeit überprüft werden. Die freie Länge der Kapillarmembranen zwischen den Einbettungen beträgt üblicherweise 120 +/- 10 mm. Die Anzahl der Kapillarmembranen ist so zu bemessen, dass unter Berücksichtigung der freien Länge und des Innendurchmessers der Kapillarmembranen eine Filtrationsfläche von ca. 30 cm$^2$ in der Prüfzelle bereitgestellt wird.

Die Prüfzelle wird mit in eine Prüfapparatur eingebunden und mit auf 25°C temperiertem ultrafiltriertem und vollentsalztem Wasser bei einem definiertem Prüfdruck (ca. 0,4 bar) durchströmt. Die während einer Messzeit von 2 min erhaltene filtrierte Wassermenge, d.h. das während der Messung erzeugte Permeat wird gravimetrisch oder volumetrisch erfasst. Vor Beginn der Messung muss die Anlage luftfrei gespült werden. Zur Bestimmung des TMF werden in der Prüfapparatur der Eingangs- und Ausgangsdruck an der Prüfzelle gemessen. Die Messung wird bei 25°C durchgeführt.

Der Transmembranfluss TMF wird nach der Formel (I)

$$TMF = \frac{V_W}{\Delta t \cdot A_M \cdot \Delta p} \quad \left[ \frac{ml}{cm^2 \cdot min \cdot bar} \right] \qquad (I)$$

ermittelt. Hierbei sind:

$V_w$ = durch die Membranprobe während der Messzeit hindurch geströmte Wasservolumen [ml]
$\Delta t$ = Messzeit [min]
$A_M$ = durchströmte Fläche der Membranprobe (üblicherweise 30 cm$^2$)
$\Delta p$ = eingestellter Druck während der Messung [bar]

Permeabilität für Wasser der Unterseite der taschenförmigen Wundauflage:

Aus dem zu prüfenden flächigen Material der Unterseite der taschenförmigen Wundauflage werden scheibenförmige Proben ausgestanzt und in einen geeigneten Probenhalter am Umfang fluiddicht so eingespannt, dass eine freie Messfläche von 17,35 cm$^2$ resultiert. Der Probenhalter befindet sich in einem Gehäuse, das von Wasser druckbeaufschlagt durchströmt werden kann. Die eingespannte Probe wird dann von auf 25°C temperiertem, vollentsalztem Wasser unter einem definierten Druck zwischen 0,1 und 0,2 bar durchströmt. Es wird das während einer Messzeit von 60 s durch die Probe hindurch geströmte Wasservolumen gravimetrisch oder volumetrisch ermittelt.

Die Permeabilität für Wasser TMF$_W$ wird nach der Formel (II)

$$TMF_W = \frac{V_W}{\Delta t \cdot A_M \cdot \Delta p} \quad \left[ \frac{ml}{cm^2 \cdot min \cdot bar} \right] \quad\quad (II)$$

ermittelt. Hierbei sind:

$V_W$ = durch die Probe während der Messzeit hindurch geströmte Wasservolumen [ml]
$\Delta t$ = Messzeit [min]
AM = durchströmte Fläche der Probe (17,35 cm$^2$)
$\Delta p$ = eingestellter Druck während der Messung [bar]

Die Erfindung wird anhand der folgenden Figuren näher erläutert, wobei durch die Figuren der Umfang der Erfindung nicht eingeschränkt wird:
Es zeigen:

Fig. 1:    Im Wundversorgungssystem einsetzbares Kapillarmembransystem mit einer Matte aus Kapillarmembranen und Versorgungsleitungen an beiden Enden der Matte.
Fig. 2:    Im Wundversorgungssystem einsetzbares Kapillarmembransystem mit einer Versorgungsleitung an einem der Mattenenden sowie U-förmig ausgebildeten Kapillarmembranenden am gegenüberliegenden Mattenende.
Fig. 3:    Querschnitt (schematisch) durch ein im Wundversorgungssystem einsetzbares taschenförmiges Wundauflagensystem.
Fig. 4:    Schnitt A - A des in Figur 3 im Querschnitt dargestellten taschenförmigen Wundauflagesystems.

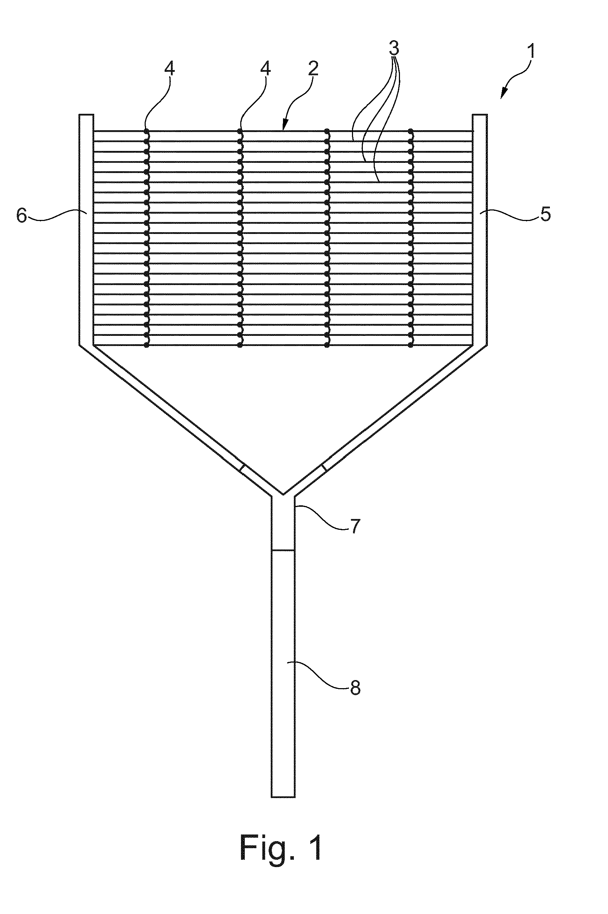

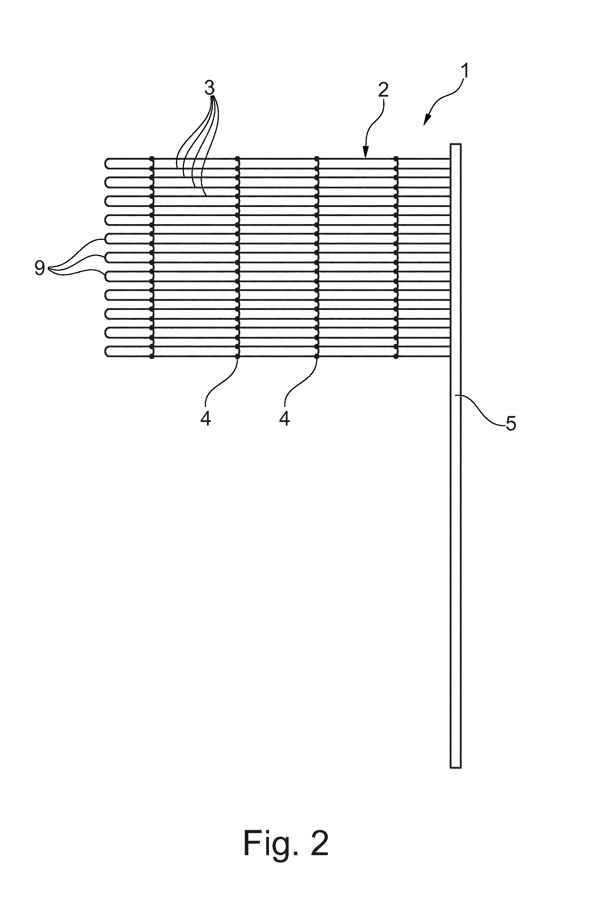

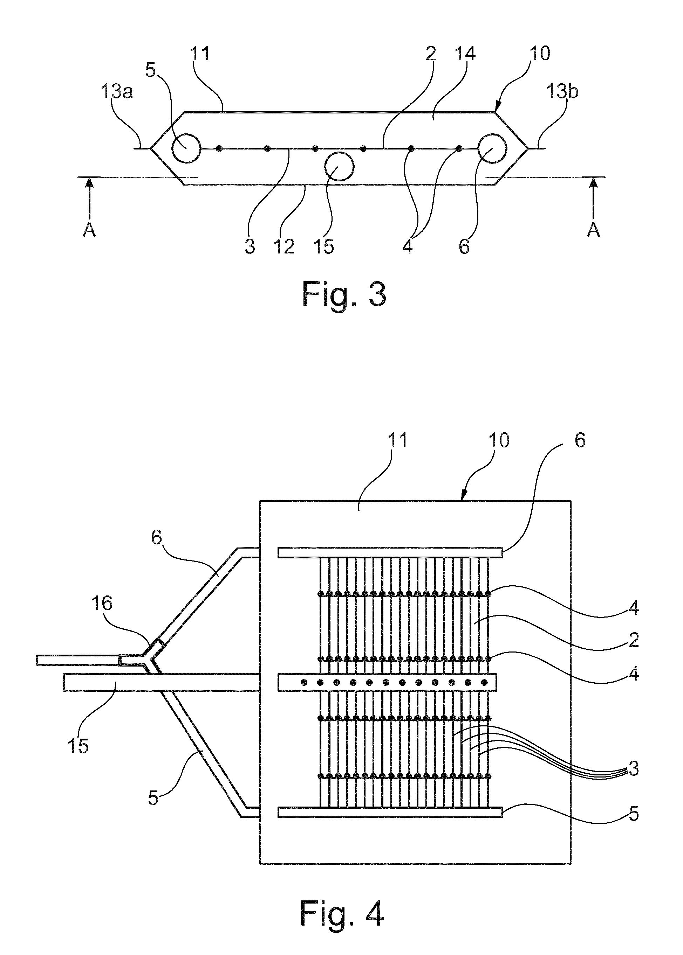

Figur 1 zeigt in der Draufsicht schematisch und nicht maßstäblich ein im erfindungsgemäßen Wundversorgungssystem 1 einsetzbares Kapillarmembransystem 2 aus Kapillarmembranen 3. Die Kapillarmembranen 3 sind mittels zueinander parallel verlaufender Verbindungselemente 4 zu einer Matte so verbunden, dass sie zueinander parallel angeordnet und zueinander auf Abstand gehalten sind. Die Kapillarmembranen 3 sind im vorliegenden Beispiel mit ihren gegenüber liegenden Enden so in Versorgungsleitungen 5, 6 eingebettet, dass zwischen den Lumen der Versorgungsleitungen 5,6 und dem Lumen der Kapillarmembranen 3 eine Fluidverbindung besteht. Die Versorgungsleitungen 5, 6 sind über ein Y-Stück 7 zu einer gemeinsamen Leitung 8 zusammengefasst. Aus diesem Aufbau ergibt sich, dass die Spülflüssigkeit bzw. die Behandlungslösung, die über die Leitung 8 zugeführt wird, auf die Versorgungsleitungen 5, 6 aufgeteilt wird und den Kapillarmembranen 3 im dead-end Modus zugeführt wird. Die Spülflüssigkeit bzw. die Behandlungslösung strömt dann über die porösen, semipermeablen Wände der Kapillarmembranen 3 aus diesen aus und wird über der Fläche des Kapillarmembransystems 2 gleichmäßig der Wunde zugeführt,
**[0052]**  Figur 2 zeigt ebenfalls schematisch und in nicht-maßstäblicher Darstellung ein Wundversorgungssystem 1, bei dem die Kapillarmembranen 3 nur mit einer Versorgungsleitung 5 verbunden sind. Die Kapillarmembranen sind an ihren beiden Enden offen und mit ihren beiden Enden in eine Versorgungsleitung 5 eingebettet. Die freien Enden 10 der Kapillarmembranen 3 sind an dem der Versorgungsleitung 5 gegenüberliegenden Ende der Matte U-förmig ausgebildet und dadurch dort geschlossen. Auf diese Weise erfolgt bei den Kapillarmembranen 3 des in Figur 3 gezeigten Kapillarmembransystems 2 die Anströmung im dead-end Modus.
Figur 3 zeigt schematisch einen Querschnitt durch eine taschenförmige Wundauflage 10, welche eine Oberseite 11 und eine Unterseite 12 aufweist, die an ihrem Rand 13a, 13b z.B. miteinander verschweißt sind, wodurch ein geschlossenes Tascheninneres 14 entsteht. Im Tascheninneren 14 ist vorliegend zur Vereinfachung der Darstellung nur ein erstes Kapillarmembransystem 2 angeordnet, welches Kapillarmembranen 3 umfasst, die über zueinander parallel verlaufende Verbindungselemente 4 vorzugsweise in Gestalt von Multifilamentgarnen miteinander verbunden und zueinander auf Abstand gehalten sind. Ein entsprechend der vorliegenden Erfindung erforderliches zweites Kapillarmembransystem kann in einer Ausführungsform oberhalb oder unterhalb des dargestellten Kapillarmembransystems 2 angeordnet sein, wobei die Versorgungsleitungen des ersten Kapillarmembransystems und des zweiten Kapillarmembransystems dann auf der gleichen Seite oder auf unterschiedlichen Seiten der taschenförmige Wundauflage 10 aus dieser herausgeführt werden können.

Die Kapillarmembranen 3 münden im vorliegenden Fall mit ihren gegenüberliegenden Enden in Versorgungsleitungen 5, 6, so dass durch die Versorgungsleitungen 5, 6 und das Kapillarmembransystem 2 Flüssigkeiten, Medien, Gase und/oder andere Stoffe hindurchleitbar sind. Die Versorgungsleitungen 5, 6 werden durch die Oberseite 11 der taschenförmigen Wundauflage 10 herausgeführt (hier nicht dargestellt).

Unterhalb des flächenförmigen Kapillarmembransystems 2 ist ein Drainageschlauch 15 angeordnet, über den z.B. sich in der Wunde ansammelndes Exsudat entfernt werden kann.

Figur 4 zeigt das in Figur 3 dargestellte Wundauflagesystem in einem Querschnitt entlang der Linie A - A. Im Prinzip handelt es sich um eine Draufsicht, von einer Position oberhalb der Unterseite 12 der taschenförmigen Wundlauflage 10 in Richtung der Oberseite 11 der taschenförmigen Wundlauflage 10. Unterhalb der Oberseite 11 , d.h. wie in Figur 3 dargestellt, zwischen Unterseite 12 und Oberseite 11 ist das Kapillarmembransystem 2 angeordnet, das aus zueinander parallelen Kapillarmembranen 3 aufgebaut ist, die über die Verbindungselemente 4 miteinander verbunden und zueinander auf Abstand gehalten sind. Die Kapillarmembranen 3 sind mit ihren gegenüberliegenden Enden in die Versorgungsleitungen 5, 6 eingebettet, so dass durch die Versorgungsleitungen 5, 6 und das Kapillarmembransystem 2 Flüssigkeiten, Medien, Gase und/oder andere Stoffe hindurchleitbar sind. Die Versorgungsleitungen 5, 6 werden durch die Oberseite 11 der taschenförmigen Wundauflage 10 durch entsprechend angepasste Öffnungen in der Oberseite 11 aus der taschenförmigen Wundauflage 10 herausgeführt und im vorliegenden Beispiel über einen Y-Verbinder 16 außerhalb der taschenförmigen Wundauflage 10 zusammengeführt. Im vorliegenden Fall wird also das Kapillarmembransystem 2 im Dead-end Modus betrieben, d.h. ein über die Versorgungsleitungen 5, 6 zugeführtes Medium wird in das Kapillarmembransystem 2 eingeleitet und tritt vollständig über die Wände der Kapillarmembranen 3 in das Tascheninnere ein.

In der Figur 2 ist auch der Drainageschlauch 15 dargestellt, der unterhalb des Kapillarmembransystems 2 angeordnet ist. Der Drainageschlauch weist in seiner Wand Perforationen auf, so dass z.B. sich in der Wunde ansammelndes Exsudat über den Drainageschlauch angesaugt und so aus der Wunde entfernt werden kann. Der Drainageschlauch 15 wird ebenfalls über eine entsprechend angepasste Öffnung in der Oberseite 11 aus der taschenförmigen Wundauflage 10 herausgeführt und ist z.B. mit einer (nicht dargestellten) Unterdruckeinheit verbindbar.

**Patentansprüche**

1. Wundversorgungssystem zur Behandlung einer Wunde, umfassend mindestens ein erstes und ein zweites flächenförmig ausgebildetes Kapillarmembransystem,

   - wobei das erste und das zweite flächenförmig ausgebildete Kapillarmembransystem mit jeweils mindestens einer Versorgungsleitung verbunden sind, so dass durch die Versorgungsleitung und das jeweilige Kapillarmembransystem Flüssigkeiten, Medien, Gase und/oder andere Stoffe durchleitbar sind,

   wobei das Wundversorgungssystem des Weiteren umfasst

   - einen ersten Entnahmebehälter mit einem Auslass, welcher über eine erste Ablaufleitung, die mindestens eine der Versorgungsleitungen des ersten Kapillarmembransystems umfasst, mit dem ersten Kapillarmembransystem lösbar verbunden ist und über den dem ersten Kapillarmembransystem eine Spülflüssigkeit zuführbar ist,
   - einen zweiten Entnahmebehälter mit einem Auslass, welcher über eine zweite Ablaufleitung, die mindestens eine der Versorgungsleitungen des zweiten Kapillarmembransystems umfasst, mit dem zweiten Kapillarmembransystem lösbar verbunden ist und über den dem zweiten Kapillarmembransystem eine Behandlungslösung zuführbar ist, sowie
   - ein Drainagesystem, welches mit einer Unterdruckeinheit koppelbar ist und über das Flüssigkeiten aus der zu behandelnden Wunde abführbar sind.

2. Wundversorgungssystem nach Anspruch 1, **dadurch gekennzeichnet, dass** das erste und/oder das zweite Kapillarmembransystem in Form einer Matte aus parallel zueinander angeordneten Kapillarmembranen ausgebildet ist, wobei die Kapillarmembranen in der Matte mittels zueinander beabstandeter und zueinander parallel verlaufender Verbindungselemente miteinander verbunden und durch die Verbindungselemente zueinander auf Abstand gehalten sind.

3. Wundversorgungssystem nach Anspruch 2, **dadurch gekennzeichnet, dass** die Kapillarmembranen mittels garnförmiger Verbindungselemente zu einer Matte verbunden sind.

4. Wundversorgungssystem nach einem oder mehreren der Ansprüche 1 bis 3, **dadurch gekennzeichnet, dass** die Kapillarmembranen des ersten bzw. des zweiten Kapillarmembransystems mit mindestens einem ihrer Enden in jeweils einer einzelnen Versorgungsleitung eingebettet sind.

5. Wundversorgungssystem nach einem oder mehreren der Ansprüche 1 bis 3, **dadurch gekennzeichnet, dass** das erste und/oder das zweite Kapillarmembransystems mit jeweils zwei Versorgungsleitungen verbunden ist, wobei die Kapillarmembranen des jeweiligen Kapillarmembransystems mit ihren gegenüberliegenden Enden in jeweils eine Versorgungsleitung eingebettet sind.

6. Wundversorgungssystem nach einem oder mehreren der Ansprüche 1 bis 5, **dadurch gekennzeichnet, dass** die Kapillarmembranen des ersten und/oder des zweiten Kapillarmembransystems einen Transmembranfluss für Wasser im Bereich von 0,01 bis 50 ml/(min·cm$^2$·bar) aufweisen.

7. Wundversorgungssystem nach einem oder mehreren der Ansprüche 1 bis 6, **dadurch gekennzeichnet, dass** die Kapillarmembranen des ersten und/oder des zweiten Kapillarmembransystems eine nominelle Pore von 0,2 $\mu$m aufweisen.

8. Wundversorgungssystem nach einem oder mehreren der Ansprüche 1 bis 7, **dadurch gekennzeichnet, dass** es des Weiteren eine taschenförmige Wundauflage umfasst, wobei die taschenförmige Wundauflage an ihrem äußeren Rand geschlossen ist und eine Oberseite, eine Unterseite und ein Tascheninneres aufweist, wobei die Unterseite und die Oberseite jeweils aus einem flächigen Material ausgebildet sind und die Unterseite permeabel für Fluide ist und wobei erstes und zweites Kapillarmembransystem im Tascheninneren angeordnet sind.

9. Wundversorgungssystem nach Anspruch 8, **dadurch gekennzeichnet, dass** das Drainagesystem im Tascheninneren angeordnet ist und über eine aus der taschenförmigen Wundauflage herausführende Verbindungsleitung mit einer Unterdruckeinheit verbindbar ist.

10. Wundversorgungssystem nach Anspruch 8, **dadurch gekennzeichnet, dass** das Drainagesystem ein Drainagekatheter ist, der über eine an seinen Querschnitt fluiddicht angepasste Durchtrittsöffnung aus der taschenförmigen Wundauflage herausführt und mit einer Unterdruckeinheit verbindbar ist.

11. Wundversorgungssystem nach einem oder mehreren der Ansprüche 8 bis 10, **dadurch gekennzeichnet, dass** die Unterseite der taschenförmigen Wundauflage aus einem vliesförmigen, flächigen Material oder einer semipermeablen, mikroporösen Flachmembran ausgebildet ist.

12. Wundversorgungssystem nach einem oder mehreren der Ansprüche 8 bis 11, **dadurch gekennzeichnet, dass** die Unterseite der taschenförmigen Wundauflage Öffnungen aufweist.

13. Wundversorgungssystem nach einem oder mehreren der Ansprüche 1 bis 12, **dadurch gekennzeichnet, dass** erstes und zweites Kapillarmembransystem identisch sind und ein einziges Versorgungs-Kapillarmembransystem ausbilden, welches mit mindestens einer Versorgungsleitung verbunden ist, und dass der erste Entnahmebehälter und der zweite Entnahmebehälter über mindestens eine Versorgungsleitung des Versorgungs-Kapillarmembransystems mit dem Versorgungs-Kapillarmembransystem verbunden sind.

14. Wundversorgungssystem nach einem oder mehreren der Ansprüche 1 bis 13, **dadurch gekennzeichnet, dass** das Verhältnis des Volumens des ersten Entnahmebehälters zum Volumen des zweiten Entnahmebehälters mindestens 5 beträgt.

15. Wundversorgungssystem nach einem oder mehreren der Ansprüche 1 bis 14, **dadurch gekennzeichnet, dass** es mindestens eine weitere Anordnung von Kapillarmembranen umfasst.

Fig. 1

Fig. 2

## Fig. 3

## Fig. 4

**Europäisches Patentamt**
**European Patent Office**
**Office européen des brevets**

# EUROPÄISCHER RECHERCHENBERICHT

**Nummer der Anmeldung**

EP 15 16 7090

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y,D | DE 10 2006 042732 B3 (GERLACH JOERG [DE]) 10. April 2008 (2008-04-10) * Absatz [0021] * ----- | 1-15 | INV. A61M1/00 A61M3/02 A61F13/00 |
| Y | US 2011/213319 A1 (BLOTT PATRICK LEWIS [GB] ET AL) 1. September 2011 (2011-09-01) * Absätze [0448], [0449]; Abbildungen 1,2 * ----- | 1-15 | |
| A | DE 103 26 747 A1 (GERLACH JOERG [DE]) 5. Januar 2005 (2005-01-05) * Ansprüche 3,5; Abbildung 1 * ----- | 1 | |
| A | WO 2009/134967 A2 (KCI LICENSING INC [US]; MCNULTY AMY [US]; KIESWETTER KRISTINE [US]) 5. November 2009 (2009-11-05) * Absatz [0053]; Abbildung 1 * ----- | 1 | |

**RECHERCHIERTE SACHGEBIETE (IPC)**

A61M
A61F

Der vorliegende Recherchenbericht wurde für alle Patentansprüche erstellt

| Recherchenort | Abschlußdatum der Recherche | Prüfer |
|---|---|---|
| Den Haag | 9. November 2015 | Lakkis, Angeliki |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 15 16 7090

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

09-11-2015

| Im Recherchenbericht angeführtes Patentdokument | Datum der Veröffentlichung | Mitglied(er) der Patentfamilie | Datum der Veröffentlichung |
|---|---|---|---|
| DE 102006042732 B3 | 10-04-2008 | DE 102006042732 B3<br>US 2009196855 A1 | 10-04-2008<br>06-08-2009 |
| US 2011213319 A1 | 01-09-2011 | US 2011213319 A1<br>US 2014107595 A1<br>US 2015080788 A1 | 01-09-2011<br>17-04-2014<br>19-03-2015 |
| DE 10326747 A1 | 05-01-2005 | DE 10326747 A1<br>US 2005015064 A1 | 05-01-2005<br>20-01-2005 |
| WO 2009134967 A2 | 05-11-2009 | CA 2722671 A1<br>EP 2282759 A2<br>TW 200950824 A<br>US 2009275884 A1<br>WO 2009134967 A2 | 05-11-2009<br>16-02-2011<br>16-12-2009<br>05-11-2009<br>05-11-2009 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

**In der Beschreibung aufgeführte Patentdokumente**

- DE 102006042732
- DE 3839567
- DE 4308850
- EP 0442147 A
- EP 1144096 A
- EP 0299381 A
- DE 2833493 A

**In der Beschreibung aufgeführte Nicht-Patentliteratur**

- HIMA-Vorschrift No. 3. Health Industry Manufacturers Association, 1982, vol. 4